# EUROPEAN PATENT APPLICATION

(11) **EP 4 316 453 A1**
(43) Date of publication of application: **07.02.2024**
(21) Application number: 22780164.4
(22) Date of filing: 17.03.2022
(51) Int. Cl.: A61K 6/35, A61K 6/60, A61K 6/70

(54) **DENTAL POLYMERIZATION KIT**

(30) Priority: 29.03.2021 JP 2021056310
(71) Applicant: Sun Medical Co., Ltd., Shiga 524-0044 (JP)
(72) Inventor: YAMADA, Yusuke, Moriyama-shi, Shiga 524-0044 (JP); KAMIMOTO, Yoshihisa, Moriyama-shi, Shiga 524-0044 (JP); INAMI, Chidzuru, Moriyama-shi, Shiga 524-0044 (JP); YOSHINAGA, Kazuhiko, Moriyama-shi, Shiga 524-0044 (JP)
(74) Representative: Hoffmann Eitle
(86) International application number: PCT/JP2022/012210
(87) International publication number: WO 2022/209944

(57) **Abstract**

A dental polymerization kit is used to polymerize a non-acidic monomer having no acidic group and having an ethylenic unsaturated group in dental treatment. The dental polymerization kit includes an A agent containing an acidic substance and a B agent containing a transition metal compound and a reducing agent and being in the form of a liquid or a paste. The transition metal compound is solid in the B agent and soluble in the acidic substance.

## Description

### TECHNICAL FIELD

The present invention relates to a dental polymerization kit.

### BACKGROUND ART

There is a conventionally-known dental curable composition that includes an A agent containing an acidic group-containing polymerizable monomer, a peroxyester (oxidizing agent), and a copper compound and a B agent containing an aromatic sulfinic acid salt (reducing agent) (for example, see Patent Document 1).

### Citation List

### Patent Document

Patent Document 1: Japanese Unexamined Patent Publication No. 2012-131717

### SUMMARY OF THE INVENTION

### PROBLEM TO BE SOLVED BY THE INVENTION

For the above dental curable composition described in Patent Document 1, the reaction of the acidic group-containing polymerizable monomer with the copper compound may adversely increase the viscosity of the A agent or cure the A agent with time.

The present invention provides a dental polymerization kit in which the gradual increase in viscosity and the gradual curing are suppressed.

### MEANS FOR SOLVING THE PROBLEM

The present invention [1] includes a dental polymerization kit used to polymerize a non-acidic monomer having no acidic group and having an ethylenic unsaturated group in dental treatment, the dental polymerization kit including: an A agent containing an acidic substance; and a B agent containing a transition metal compound and a reducing agent and being in the form of a liquid or a paste, wherein the transition metal compound is solid in the B agent and soluble in the acidic substance.

The present invention [2] includes the dental polymerization kit described in the above-described [1], wherein the A agent further contains an oxidizing agent.

The present invention [3] includes the dental polymerization kit described in the above-described [1] or [2], wherein the acidic substance is an acidic monomer having an acidic group and an ethylenic unsaturated group.

The present invention [4] includes the dental polymerization kit described in any one of the above-described [1] to [3], wherein at least one of the A agent and the B agent contains the non-acidic monomer.

The present invention [5] includes the dental polymerization kit described in any one of the above-described [1] to [4], wherein a ratio of the transition metal compound in the B agent is 0.0001% by mass or more.

### EFFECTS OF THE INVENTION

In the dental polymerization kit of the present invention, the acidic substance is contained in the A agent and the transition metal compound is contained in the B agent. This can prevent the reaction of the acidic substance with the transition metal compound.

Further, the transition metal compound is solid in the B agent. This can suppress the reaction of the transition metal compound with the reducing agent in the B agent.

As a result, the gradual increase in viscosity of the A agent and B agent or the gradual curing of them are suppressed.

### DESCRIPTION OF THE EMBODIMENTS

### 1. Two-component Self-adhesive Resin Cement

A two-component self-adhesive resin cement as an example of the dental polymerization kit is used, for example, to bond a prosthetic to the decayed part of a tooth in dental treatment. The two-component self-adhesive resin cement includes an A agent and a B agent. The two-component self-adhesive resin cement is used, for example, by mixing the A agent and the B agent together immediately before the use and applying the obtained mixture. Alternatively, the two-component self-adhesive resin cement is used, for example, by applying the A agent and thereafter applying the B agent.

The term "self-adhesive" means that the resin cement has sufficient adhesiveness to a tooth structure without applying a pretreatment agent to the tooth structure.

### (1) A agent

The A agent contains an acidic substance. If necessary, the A agent contains a non-acidic monomer, a filler, an oxidizing agent, and a photopolymerization initiator. When the B agent does not contain a non-acidic monomer and a filler, the A agent necessarily contains a non-acidic monomer and a filler. In the present embodiment, the A agent is in the form of a liquid or a paste.

### (1-1) Acidic substance

The acidic substance dissolves the transition metal compound in the B agent when the A agent and the B agent is mixed together. The transition metal compound is described below. Examples of the acidic substance include an acidic monomer, an anhydride of an acidic monomer, an inorganic acid, and an organic acid. The organic acid does not include an acidic monomer and an anhydride of an acidic monomer. As the acidic substance, an acidic monomer and an anhydride of an acidic monomer are preferable.

The acidic monomer has an acidic group and an ethylenic unsaturated group.

Examples of the acidic group inclues a carboxy group, a phosphoric acid group, a thiophosphoric acid group, and a sulfo group. The acidic monomer may contain one type of acidic groups or two or more types of acidic groups.

Examples of the ethylenic unsaturated group include an acryloyl group, a methacryloyl group, and a vinyl group.

In the description below, the terms "acryloyl" and "methacryloyl" are collectively referred to as "(meth)acryloyl". Furhter, the terms "acrylate" and "methacrylate" are collectively referred to as "(meth)acrylate".

Examples of the acidic monomer having a carboxy group include an aromatic carboxylic acid derivative, an aliphatic carboxylic acid derivative, and an amino acid derivative.

Examples of the aromatic carboxylic acid derivative include a benzoic acid derivative, a salicylic acid derivative, a phthalic acid derivative, a trimellitic acid derivative, a pyromellitic acid derivative, a naphthalene tricarboxylic acid derivative, a benzophenone tetracarboxylic acid derivative, and a biphenyl tetracarboxylic acid derivative.

Examples of the benzoic acid derivative include 4-vinylbenzoic acid, 2-(meth)acryloyloxy benzoic acid, 3-(meth)acryloyloxy benzoic acid, 4-(meth)acryloyloxy benzoic acid, N-(meth)acryloyl-4-aminobenzoic acid, and N-(meth)acryloyl-5-aminobenzoic acid.

Examples of the salicylic acid derivative include 4-(meth)acryloylaminosalicylic acid, and 5-(meth)acryloylaminosalicylic acid.

Examples of the phthalic acid derivative include 4-[(2-hydroxy-3-(meth)acryloyl oxypropyl)amino]phthalic acid, 3-[N-methyl-N-(2-hydroxy-3-(meth)acryloyl oxypropyl)amino]phthalic acid, and 4-(N-methyl-N-(2-hydroxy-3-(meth)acryloyl oxypropyl)amino]phthalic acid.

Examples of the trimellitic acid derivative include 4-(meth)acryloyloxyalkyl trimellitic acid, 4-[2-hydroxy-3-(meth)acryloyl oxy]butyl trimellitic acid, 2-3-bis(3,4-dicarboxy benzoyloxy)propyl (meth)acrylate, and 2-(3,4-dicarboxy benzoyloxy)-1,3-di(meth)acryloyl oxy propane. Examples of the 4-(meth)acryloyloxyalkyl trimellitic acid include 4-(meth)acryloyl oxymethyl trimellitic acid, 4-(meth)acryloyl oxyethyl trimellitic acid, and 4-(meth)acryloyl oxybutyl trimellitic acid.

Examples of the pyromellitic acid derivative include 1,4-di(meth)acryloyl oxyethylpyromellitic acid, and a reaction product of 2-hydroxyethyl (meth)acrylate and a dianhydride of pyromellitic acid.

Examples of the naphthalene tricarboxylic acid derivative include 6-(meth)acryloyl oxyethylnaphthalene-1,2,6-tricarboxylic acid.

Examples of the benzophenone tetracarboxylic acid derivative include a reaction product of 2-hydroxyethyl (meth)acrylate and a dianhydride of 3,3',4,4'-benzophenone tetracarboxylic acid.

Examples of the biphenyl tetracarboxylic acid derivative include a reaction product of 2-hydroxyethyl (meth)acrylate and a dianhydride of 3,3',4,4'-biphenyl tetracarboxylic acid.

Examples of the aliphatic carboxylic acid derivative include maleic acid, a reaction product of 2-hydroxyethyl (meth)acrylate and maleic anhydride, and 11-(meth)acryloyl oxy-1,1-undecane dicarboxylic acid.

Examples of the amino acid derivative include N,O-di(meth)acryloyl oxy tyrosine, O-(meth)acryloyl oxy tyrosine, N-(meth)acryloyl oxy tyrosine, N-(meth)acryloyl oxy phenylalanine, a reaction product of N-phenylglycine and glycidyl (meth)acrylate, and a reaction product of N-tolyl glycine and glycidyl (meth)acrylate.

As the acidic monomer having a carboxy group, an aromatic carboxylic acid derivative is preferable, a trimellitic acid derivative is more preferable, 4-(meth)acryloyloxyalkyl trimellitic acid is more preferable, 4-(meth)acryloyl oxyethyl trimellitic acid is more preferable, and 4-methacryloyl oxyethyl trimellitic acid (4-MET) is more preferable.

Examples of the acidic monomer having a phosphoric acid group include an aliphatic phosphate and an aromatic phosphate.

Examples of the aliphatic phosphate include (meth)acryloyl oxyalkyl acid phosphate, and bis[(meth)acryloyl oxyalkyl] acid phosphate.

Examples of the (meth)acryloyl oxyalkyl acid phosphate include 2-(meth)acryloyl oxyethyl acid phosphate, 2-(meth)acryloyl oxypropyl acid phosphate, 3-(meth)acryloyl oxypropyl acid phosphate, 4-(meth)acryloyl oxybutyl acid phosphate, 6-(meth)acryloyl oxyhexyl acid phosphate, 8-(meth)acryloyl oxy octyl acid phosphate, 10-(meth)acryloyl oxy decyl acid phosphate, and 12-(meth)acryloyl oxydodecyl acid phosphate.

Examples of the bis[(meth)acryloyl oxyalkyl] acid phosphate include bis[2-(meth)acryloyl oxyethyl] acid phosphate, bis[2-(meth)acryloyl oxypropyl] acid phosphate, and bis[3-(meth)acryloyl oxypropyl] acid phosphate.

Examples of the aromatic phosphate include 2-(meth)acryloyl oxyethylphenyl acid phosphate and 2-(meth)acryloyl oxyethyl-p-methoxyphenyl acid phosphate.

As the acidic monomer having a phosphoric acid group, aliphatic phosphate is preferable, (meth)acryloyl oxyalkyl acid phosphate is more preferable, 10-(meth)acryloyl oxy decyl acid phosphate is more preferable, and 10-methacryloyl oxy decyl acid phosphate (MDP) is more preferable.

Examples of the acidic monomer having a thiophosphoric acid group include a compound obtained by replacing the phosphoric acid group of the above-described acidic monomer having a phosphoric acid group with a thiophosphoric acid group.

Examples of the inorganic acid include muriatic acid, phosphoric acid, sulfuric acid, nitric acid, boric acid, and hypochlorous acid.

Examples of the organic acid include monocarboxylic acid, dicarboxylic acid, and polyvalent carboxylic acid. Examples of the monocarboxylic acid include formic acid, acetic acid, and propionic acid. Examples of the dicarboxylic acid include oxalic acid, malonic acid, succinic acid, glutaric acid, and phthalic acid. Examples of the polyvalent carboxylic acid include malic acid, tartaric acid, and citric acid.

The ratio of the acidic substance in the A agent is not limited as long as the transition metal compound is sufficiently dissolved when the A agent and the B agent are mixed together. The term "sufficiently" means "to the degree that the oxidation-reduction reaction of the oxidizing agent with the reducing agent can be accelerated". The ratio of the acidic substance in the A agent is, for example, 0.1% by mass or more, preferably 0.2% by mass or more, more preferably 0.5% by mass or more, and, for example, 50% by mass or less, preferably 30% by mass or less, more preferably 20% by mass or less.

When the acidic substance is an acidic monomer and at least one of the A agent and the B agent contains a non-acidic monomer, the ratio of the acidic monomer to the total amount of the non-acidic monomer in the A agent and B agent is, for example, 1% by mass or more, preferably 2% by mass or more, more preferably 5% by mass or more, and, for example, 50% by mass or less, preferably 30% by mass or less, more preferably 20% by mass or less.

### (1-2) Non-acidic Monomer

The non-acidic monomer does not have the above-described acidic group and has the above-described ethylenic unsaturated group.

The non-acidic monomers are classified into non-acidic monomers having hydroxyl groups, non-acidic monomers having urethane bonds, and non-acidic monomers having no hydroxyl group and no urethane bond.

Examples of the non-acidic monomer having a hydroxyl group include mono (meth)acrylate and di(meth)acrylate.

Examples of the mono(meth)acrylate having a hydroxyl group include 2-hydroxyethyl (meth)acrylate, 3-hydroxypropyl (meth)acrylate, 6-hydroxyhexyl (meth)acrylate, and dihydroxypropylmono (meth)acrylate. As the mono(meth)acrylate having a hydroxyl group, 2-hydroxyethyl (meth)acrylate is preferable, and 2-hydroxyethylmethacrylate (HEMA) is more preferable.

Examples of the di(meth)acrylate having a hydroxyl group include a bisphenol A derivative. Examples of the bisphenol A derivative having a hydroxyl group include 2,2-bis[4-(3-(meth)acryloyl oxy-2-hydroxypropoxy)phenyl] propane (Bis-GMA).

Examples of the non-acidic monomer having a urethane bond include [2,2,4-trimethylhexamethylenebis(2-carbamoyl oxyethyl)]di(meth)acrylate (UDMA).

Examples of the non-acidic monomer having no hydroxyl group and no urethane bond include mono(meth)acrylate and di(meth)acrylate.

Examples of the mono(meth)acrylate having no hydroxyl group and no urethane bond include alkyl (meth)acrylate. Examples of the alkyl (meth)acrylate include methyl (meth)acrylate, ethyl (meth)acrylate, and butyl (meth)acrylate.

Examples of the di(meth)acrylate having no hydroxyl group and no urethane bond include an alkane diol di(meth)acrylate and a bisphenol A derivative.

Examples of the alkane diol di(meth)acrylate include ethylene glycol di(meth)acrylate, propylene glycol di(meth)acrylate, and neopentyl glycol di(meth)acrylate. As the alkane diol di(meth)acrylate, neopentyl glycol dimethacrylate (NPGDMA) is preferable.

Examples of the bisphenol A derivative having no hydroxyl group and no urethane bond include ethylene oxide-modified bisphenol A di(meth)acrylate.

These non-acidic monomers can be used singly or in combination of two or more.

Among the above-described non-acidic monomers, the A agent contains the non-acidic monomer having a hydroxyl group and the non-acidic monomer having a urethane bond and does not contain the non-acidic monomer having no hydroxyl group and no urethane bond. Alternatively, the A agent contains the non-acidic monomer having no hydroxyl group and no urethane bond and does not contain the non-acidic monomer having a hydroxyl group and the non-acidic monomer having a urethane bond.

The ratio of the non-acidic monomer in the A agent is, for example, 5% by mass or more, preferably 10% by mass or more, more preferably 15% by mass or more, and, for example, 95% by mass or less, preferably 90% by mass or less, more preferably 85% by mass or less.

### (1-3) Filler

Examples of the filler include a conventionally-known filler that can be used for detal materials. Specifically, examples of the filler include silica, silica alumina, alumina, alumina quartz, glass, titania, zirconia, and ytterbium fluoride. Examples of the silica include fumed silica. Examples of the glass include barium glass. The filler may be hydrophobized or hydrophilized by processing a surface of the filler with, for example, a coupling agent.

The ratio of the filler in the A agent is, for example, 0.5% by mass or more, preferably 5% by mass or more, more preferably 15% by mass or more, and, for example, 97% by mass or less, preferably 95% by mass or less, more preferably 90% by mass or less, more preferably 85% by mass or less, more preferably 75% by mass or less.

### (1-4) Oxidizing Agent

The oxidizing agent oxidizes the reducing agent in the mixture of the A agent and the B agent when the A agent and the B agent are mixed together. In other words, the oxidizing agent is reduced with the reducing agent when the A agent and the B agent are mixed together. This oxidation-reduction reaction produces radicals. At the time, the transition metal compound functions as an oxidation-reduction reaction accelerator. The transition metal compound is described below.

Examples of the oxidizing agent include an organic peroxide and an inorganic peroxide.

Examples of the organic peroxide include diacyl peroxide, peroxyester, dialkyl peroxide, peroxyketal, ketone peroxide, and hydroperoxide.

Examples of the diacyl peroxide include benzoyl peroxide, diacetyl peroxide, 2,4-dichlorobenzoyl peroxide, decanoyl peroxide, 3,3,5-trimethylhexanoyl peroxide, lauroyl peroxide, and m-toluoyl peroxide.

Examples of the peroxyester include t-butylperoxybenzoate, bis-t-butylperoxyisophthalate, 2,5-dimethyl-2,5-bis(benzoylperoxy)hexane, t-butylperoxy-2-ethylhexanoate, and t-butylperoxyisopropyl carbonate.

Examples of the dialkyl peroxide include dicumyl peroxide, di-t-butyl peroxide, 1,3-bis(t-butylperoxyisopropyl)benzene, and 2,5-dimethyl-2,5-di(t-butylperoxy)-3-hexane.

Examples of the peroxyketal include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and 4,4-bis(t-butylperoxy)valerate-n-butyl ester.

Examples of the ketone peroxide include methylethylketone peroxide.

Examples of the hydroperoxide include cumene hydroperoxide, t-butylhydroperoxide, t-amyl hydroperoxide, p-diisopropylbenzenehydroperoxide, 1,1,3,3-tetramethylbutylhydroperoxide, and p-methanehydroperoxide.

As the organic peroxide, peroxyester and hydroperoxide are preferable, t-butylperoxybenzoate (BPB) and cumene hydroperoxide (CUP) is more preferable.

Examples of the inorganic peroxide include a persulfate compound, a superphosphate compound, a perchlorate compound, and a hydrogen peroxide derivative.

Examples of the persulfate compound include sodium persulfate, potassium persulfate, calcium persulfate, magnesium persulfate, and ammonium persulfate.

Examples of the superphosphate compound include sodium superphosphate, potassium superphosphate, calcium superphosphate, magnesium superphosphate, and ammonium superphosphate.

Examples of the perchlorate compound include sodium perchlorate, potassium perchlorate, calcium perchlorate, magnesium perchlorate, and ammonium perchlorate.

Examples of the hydrogen peroxide derivative include hydrogen peroxide and urea peroxide.

As the inorganic peroxide, persulfate compound is preferable, and sodium persulfate and potassium persulfate are more preferable.

The A agent may contain one type of oxidizing agents or a plurality types of oxidizing agents.

The ratio of the oxidizing agent in the A agent is, for example, 0.01% by mass or more, preferably 0.05% by mass or more, more preferably 0.1% by mass or more, and, for example, 10% by mass or less, preferably 7% by mass or less, more preferably 5% by mass or less.

### (1-5) Photopolymerization Initiator

The photopolymerization initiator produces radicals by reciving light.

Examples of the photopolymerization initiator include α-diketone, ketal, and thioxanthone. Examples of the α-diketone include camphorquinone. Examples of the ketal include benzyl dimethyl ketal, and benzyl diethyl ketal. Examples of the thioxanthone include 2-chlorothioxanthone and 2,4-diethylthioxanthone.

As the photopolymerization initiator, α-diketone is preferable, and camphorquinone (CQ) is more preferable.

The ratio of the photopolymerization initiator in the A agent is, for example, 0.001% by mass or more, preferably 0.01% by mass or more, more preferably 0.1% by mass or more, more preferably 0.2% by mass or more, and, for example, 5% by mass or less, preferably 3% by mass or less, more preferably 1% by mass or less, more preferably 0.5% by mass or less.

### (1-6) Another Additive

If necessary, the A agent may contain a polymerization inhibitor, a thickening agent, a coupling agent, an ultraviolet absorber, a fluorescing agent, a pigment, and a solvent. Examples of the polymerization inhibitor include dibutylhydroxytoluene (BHT).

### (2) B Agent

The B agent contains a transition metal compound and a reducing agent. If necessary, the B agent contains a non-acidic monomer and a filler. When the A agent does not contain a non-acidic monomer and a filler, the B agent necessarily contains a non-acidic monomer and a filler. The B agent is in the form of a liquid or a paste.

### (2-1) Transition Metal Compound

At leat a part of the transition metal compound is soluble in the acidic substance. The whole of the transition metal compound is preferably soluble in the acidic substance. The transition metal compound is dissolved in the acidic substance when the A agent and the B agent are mixed together, and the dissolution produces transition metal ions. The transition metal ions are chemically reacted with the reducing agent, the dissolved oxygen, or the oxidizing agent, thereby producing radicals. The produced radicals polymerize the non-acidic monomer.

The transition metal compound contains a transition metal atom in 4th period of the periodic table. The periodic table is the IUPAC Periodic Table of the Elements (version date 1 December 2018).

Specifically, examples of the transition metal atom include scandium (Sc), titanium (Ti), vanadium (V), chromium (Cr), manganese (Mn), iron (Fe), cobalt (Co), nickel (Ni), copper (Cu), zinc (Zn), and gallium (Ga). As the transition metal atom, titanium (Ti), vanadium (V), chromium (Cr), manganese (Mn), iron (Fe), cobalt (Co), nickel (Ni), copper (Cu), and zinc (Zn) are preferable, and vanadium (V), chromium (Cr), manganese (Mn), iron (Fe), cobalt (Co), nickel (Ni), copper (Cu), and zinc (Zn) are more preferable.

Examples of the transition metal compound include an oxide of a transition metal, hydroxide, halide, carboxylate, sulfate, nitrate, carbonate, and a complex. Examples of the halide include chloride and bromide. Examples of the carboxylate include formate, acetate, oleic acid, acrylate, gluconate, phthalate, and citrate.

A complex of a transition metal has a transition metal atom as the central metal and ligands coordinated with the transition metal atom.

Examples of the ligands include diketone ligands, amine ligands, thiol ligands, urea ligands, and thiourea ligands. Examples of the diketone ligands include acetylacetonate. Examples of the amine ligands include alkanediamine, benzimidazole, and benzothiazole. Examples of the thiol ligands include mercaptoethanol, mercaptotriazole, mercaptobenzothiazole, and mercaptobenzoimidazol. Examples of the urea ligands include urea and ethyleneurea. Examples of the thiourea ligands include thiourea, acetylthiourea, pyridylthiourea, and ethylenethiourea.

Specifically, examples of the transition metal compound include a copper compound, an iron compound, a cobalt compound, a chromium compound, and a vanadium compound.

Examples of the copper compound include copper(II) hydroxide, copper(I) chloride, copper(II) chloride, copper(I) bromide, copper(II) bromide, copper(II) formate, copper(I) acetate, copper(II) acetate, copper(II) sulfate, copper(II) nitrate, copper(I) iodide, basic copper(II) carbonate, copper(I) oxide, copper(II) oxide, metal copper, copper(II) oleate, copper(II) acrylate, copper(II) gluconate, copper(II) phthalate, copper(II) citrate, copper (II) acetylacetonate, mercaptobenzothiazole copper, 1,3- propanediamine copper, benzimidazole copper, benzothiazole copper, and thiourea copper.

Examples of the iron compound include iron(II) hydroxide, iron(III) oxide-hydroxide, iron(II) chloride, iron(III) chloride, iron(I) bromide, iron(II) bromide, iron(II) acetate, iron(III) acetate, iron(II) carbonate, iron(II) oxide, iron(III) oxide, iron(II, III) oxide, metallic iron, iron(II) sulfate, iron(III) sulfate, iron(III) acetylacetonate, iron(III) citrate, and iron(II) gluconate.

Examples of the cobalt compound include cobalt(II) hydroxide, cobalt(II) chloride, cobalt(II) acetate, cobalt(II) bromide, basic cobalt carbonate, cobalt(II) sulfate, cobalt(II) oxide, cobalt(III) oxide, cobalt(II, III) oxide, metallic cobalt, cobalt(II) nitrate, and cobalt(III) acetylacetonate.

Examples of the vanadium compound include vanadium(III) chloride, vanadium(III) bromide, metallic vanadium, vanadium(II) oxide, vanadium(III) oxide, vanadium(V) oxide, vanadium (IV) oxy acetylacetonate, vanadium(III) acetylacetonate, oxovanadium(IV) oxalate, vanadium(IV) oxide stearate, vanadium(IV) oxide sulfate, ammonium vanadate(V), and sodium orthovanadate (V).

The transition metal compound is solid in the B agent. That is to say, the B agent contains the components in the B agent and the particulates of the transition metal compound insoluble in the moisture content obtained by the moisture absorption during storage. The amount of the transition metal compound dissolved with respect to 100 g of water at 20°C and a pressure of one atmosphere is, for example, 5 g or less, preferably 4 g or less, more preferably 3 g or less. The average particle size of the particulates is not limited. However, if the particulates have excessively large particle sizes, they tend to easily settle. If the particulates have excessively small particle sizes, they cause an excessively large specific surface area. These decrease the amount of the particulates that can be dispersed. Thus, the average particle size is, for example, 0.01 µm or more, preferably 0.1 µm or more, more preferably 1 µm or more, and, for example, 500 µm or less, preferably 100 µm or less, more preferably 50 µm or less.

These transition metal compounds can be used singly or in combination of two or more.

The ratio of the transition metal compound in the B agent is, for example, 0.0001% by mass or more, preferably 0.0005% by mass or more, more preferably 0.001% by mass or more, and, for example, 1% by mass or less, preferably 0.5% by mass or less, more preferably 0.3% by mass or less.

The ratio of the transition metal compound to the reducing agent is, for example, 0.001% by mass or more, preferably 0.005% by mass or more, more preferably 0.01% by mass or more, and, for example, 10% by mass or less, preferably 5% by mass or less, more preferably 3% by mass or less.

When the A agent and the B agent is mixed together in a volume ratio of 1: 1, the ratio of the transition metal compound to the oxidizing agent in the A agent is, for example, 0.001% by mass or more, preferably 0.005% by mass or more, more preferably 0.01% by mass or more, and, for example, 10% by mass or less, preferably 5% by mass or less, more preferably 3% by mass or less.

### (2-2) Reducing Agent

The reducing agent reduces the transition metal ions when the A agent and the B agent are mixed together. In other words, the reducing agent is oxidized by the transition metal ions when the A agent and the B agent are mixed together. The oxidization of the reducing agent by the transition metal ions produces radicals.

Examples of the reducing agent include an aromatic amine compound, sulfinic acid or its salts, a thiourea compound, a phosphorus acid compound, and a sulfurous acid compound.

Examples of the aromatic amine compound include an aromatic tertiary amine and an aromatic amino acid.

Examples of the aromatic tertiary amine include N,N-dialkyl toluidine, N,N-bis(hydroxyalkyl)toluidine, N,N-dialkyl aniline, and dialkyl aminobenzoic acid ester.

Examples of the N,N-dialkyl toluidine include N,N-dimethyl-p-toluidine (DMPT), and N,N-diethyl-p-toluidine.

Examples of the N,N-bis(hydroxyalkyl)toluidine include N,N-bis(2-hydroxyethyl)-p-toluidine, and N,N-bis(2-hydroxypropyl)-p-toluidine.

Examples of the N,N-dialkyl aniline include N,N-dimethyl aniline and N,N-diethyl aniline.

Examples of the dialkyl aminobenzoic acid ester include methyl 4-(dimethylamino)benzoate, ethyl 4-(dimethylamino)benzoate (EDAB), 2-butoxyethyl 4-(dimethylamino)benzoate, and isoamyl 4-(dimethylamino)benzoate.

Examples of the aromatic amino acid include N-phenyl glycine, sodium N-phenyl glycine (SPG), and potassium N-phenyl glycine.

These aromatic amine compounds can be used singly or in combination of two or more.

Examples of the sulfinic acid and its salts include aromatic sulfinic acid and its salts. Examples of the aromatic sulfinic acid include benzene sulfinic acid, o-toluenesulfinate, p-toluenesulfinate, ethylbenzenesulfinic acid, decylbenzenesulfinic acid, dodecylbenzenesulfinic acid, chlorobenzenesulfinic acid, fluorobenzenesulfinic acid, and naphthalenesulfinic acid.

Examples of a salt of aromatic sulfinic acid include lithium benzenesulfinate, sodium benzenesulfinate, potassium benzenesulfinate, magnesium benzenesulfinate, calcium benzenesulfinate, strontium benzenesulfinate, barium benzenesulfinate, benzenesulfinic acid butylamine salt, benzenesulfinic acid aniline salt, benzenesulfinic acid toluidine salt, benzenesulfinic acid phenylene diamine salt, benzenesulfinic acid diethylamine salt, benzenesulfinic acid diphenylamine salt, benzenesulfinic acid triethylamine salt, benzenesulfinic acid tributylamine salt, benzenesulfinic acid ammonium salt, benzenesulfinic acid tetramethyl ammonium, benzenesulfinic acid trimethylbenzyl ammonium, lithium o-toluenesulfinate, sodium o-toluenesulfinate, potassium o-toluenesulfinate, calcium o-toluenesulfinate, o-toluenesulfinate cyclohexylamine salt, o-toluenesulfinate aniline salt, o-toluenesulfinate ammonium salt, o-toluenesulfinate tetraethyl ammonium, lithium p-toluenesulfinate, sodium p-toluenesulfinate (STS), potassium p-toluenesulfinate, calcium p-toluenesulfinate, barium p-toluenesulfinate, p-toluenesulfinate ethylamine salt, p-toluenesulfinate butylamine salt, p-toluenesulfinate toluidine salt, p-toluenesulfinate N-methylaniline salt, p-toluenesulfinate pyridine salt, p-toluenesulfinate ammonium salt, p-toluenesulfinate tetramethyl ammonium, p-toluenesulfinate tetraethyl ammonium, p-toluenesulfinate tetrabutyl ammonium, sodium β-naphthalenesulfinate, strontium β-naphthalenesulfinate, β-naphthalenesulfinate triethylamine, β-naphthalenesulfinate N-methyl toluidine, ammonium β-naphthalenesulfinate, β-naphthalenesulfinate trimethyl benzyl ammonium, lithium p-chlorobenzenesulfinate, sodium p-chlorobenzenesulfinate, potassium p-chlorobenzenesulfinate, calcium p-chlorobenzenesulfinate, barium p-chlorobenzenesulfinate, p-chlorobenzenesulfinate ethylamine salt, p-chlorobenzenesulfinate butylamine salt, p-chlorobenzenesulfinate toluidine salt, p-chlorobenzenesulfinate N-methylaniline salt, p-chlorobenzenesulfinate pyridine salt, p-chlorobenzenesulfinate ammonium salt, p-chlorobenzenesulfinate tetramethylammonium, p-chlorobenzenesulfinate tetraethylammonium, and p-chlorobenzenesulfinate tetrabutylammonium.

Examples of the thiourea compound include thiourea, methyl thiourea, ethyl thiourea, n-propyl thiourea, isopropyl thiourea, cyclohexyl thiourea, benzyl thiourea, phenyl thiourea, acetyl thiourea, benzoyl thiourea, adamanthyl thiourea, (2-pyridyl)thiourea (PTU), 1-(2-tetrahydrofurfuryl)-2-thiourea, N,N'-dimethylthiourea, N,N'-diethylthiourea, N,N'-di-n-propylthiourea, N,N'-di-isopropylthiourea, N,N'-dicyclohexylthiourea, N,N'-diphenylthiourea, trimethylthiourea (TMTU), triethylthiourea, tri-n-propylthiourea, triisopropylthiourea, tricyclohexylthiourea, tetramethylthiourea, tetraethylthiourea, tetra-n- propylthiourea, tetraisopropylthiourea, tetracyclohexylthiourea, ethylenethiourea, and 4,4-dimethylethylenethiourea.

Examples of the phosphorus acid compound include an inorganic phosphorus acid compound and an organic phosphorus acid compound.

Examples of the inorganic phosphorus acid compound include calcium hypophosphite, and sodium phosphite.

Examples of the organic phosphorus acid compound include diethyl phosphite, dibutyl phosphite, diisopropyl phosphite, di-n-propy phosphite, triphenyl phosphite, triallyl phosphite, diethyl phosphite, dibutyl phosphite, diisopropyl phosphite, di-n-propy phosphite, triphenyl phosphite, and triallyl phosphite.

Examples of the sulfurous acid compound include an inorganic sulfurous acid compound and an organic sulfurous acid compound.

Examples of the inorganic sulfurous acid compound include lithium sulfite, sodium sulfite, potassium sulfite, calcium sulfite, and sodium hydrogen sulfite.

Examples of the organic sulfurous acid compound include diethyl sulfite, di-n-propyl sulfite, diisopropyl sulfite, glycol sulfite, 1-3-propylene sulfite, and diallyl sulfite.

Examples of the thiosulfuric acid compound include sodium thiosulfate, calcium thiosulfate, potassium thiosulfate, and magnesium thiosulfate.

These reducing agents can be used singly or in combination of two or more.

As the reducing agent, a combination of an aromatic amine compound/sulfinic acid or its salts/a thiourea compound is preferable, a combination of aromatic tertiary amine/aromatic sulfinic acid or its salts/alkylthiourea is more preferable, a combination of dialkylaminobenzoic acid ester/p-toluenesulfinate or a combination of sodium p-toluenesulfinate/trialkylthiourea is more preferable, and a combination of 4-dimethylaminobenzoic acid ethyl/p-toluenesulfinic acid sodium salt/trimethylthiourea is more preferable.

The ratio of the reducing agent in the B agent is, for example, 0.01% by mass or more, preferably 0.1% by mass or more, more preferably 0.5% by mass or more, and, for example 30% by mass or less, preferably 20% by mass or less, more preferably 10% by mass or less.

### (2-3) Non-acidic Monomer

Examples of the non-acidic monomer in the B agent include the same monomer as the non-acidic monomer in the A agent.

When the A agent contains a non-acidic monomer having a hydroxyl group and a non-acidic monomer having a urethane bond among the above-described non-acidic monomers, the B agent contains a non-acidic monomer having no hydroxyl group and no urethane bond. When the A agent contains a non-acidic monomer having no hydroxyl group and no urethane bond among the above-described non-acidic monomers, the B agent contains a non-acidic monomer having a hydroxyl group and a non-acidic monomer having a urethane bond.

The ratio of the non-acidic monomer in the B agent is not limited. The ratio of the non-acidic monomer in the B agent is, for example, 5% by mass or more, preferably 10% by mass or more, more preferably 15% by mass or more, and, for example, 99% by mass or less, preferably 90% by mass or less, more preferably 85% by mass or less.

### (2-4) Filler

The description of the filler in the B agent is the same as that of the filler in the A agent.

The ratio of the filler in the B agent is, for example, 10% by mass or more, preferably 20% by mass or more, more preferably 30% by mass, and, for example, 97% by mass or less, preferably 95% by mass or less, more preferably 90% by mass or less.

### (2-5) Photopolymerization Initiator

The description of the photopolymerization initiator in the B agent is the same as that of the photopolymerization initiator in the A agent.

The ratio of the photopolymerization initiator in the B agent is, for example, 0.001% by mass or more, preferably 0.005% by mass or more, more preferably 0.01% by mass or more, and, for example, 2% by mass or less, preferably 1% by mass or less, more preferably 0.5% by mass or less.

### (2-6) Another Additive

If necessary, the B agent may contain a polymerization inhibitor, a thickening agent, a solvent, a coupling agent, an ultraviolet absorber, a fluorescing agent, and a pigment. The descriptions of the polymerization inhibitor, thickening agent, solvent, coupling agent, ultraviolet absorber, fluorescing agent, and pigment in the B agent are the same as those of the polymerization inhibitor, thickening agent, solvent, coupling agent, ultraviolet absorber, fluorescing agent, and pigment in the A agent.

### 2. Reaction Mechanism

The reaction mechanism of the above-described two-component self-adhesive resin cement is described next.

To use the two-component self-adhesive resin cement, the A agent and the B agent are mixed together or applied in layers.

When the A agent and the B agent are mixed together, or the A agent and the B agent are brought into contact with each other, the transition metal compound in the B agent is dissolved in the acidic substance in the A agent. The dissolution of the transition metal compound in the acidic substance produces transition metal ions. The transition metal ions are chemically reacted with the reducing agent, the dissolved oxygen, and the oxidizing agent, thereby producing radicals. The produced radicals polymerize the non-acidic monomers. That is to say, the dental polymerization kit is used to polymerize the non-acidic monomers in dental treatment.

### 3. Operation and Effect

In the two-component self-adhesive resin cement, the acidic substance is contained in the A agent, and the transition metal compound is contained in the B agent. This can prevent the reaction of the acidic substance with the transition metal compound.

Further, the transition metal compound is solid in the B agent. This can prevent the reaction of the transition metal compound with the reducing agent in the B agent.

As a result, the increase in viscosity of the A agent and B agent with time or the curing of them with time are suppressed.

### 4. Another embodiment

Another embodiment of the dental polymerization kit is described below.

(1) The dental polymerization kit may contain a one-component dental pretreatment agent and a one-component dental resin cement. The one-component dental pretreatment agent is used, for example, to improve the adhesiveness of the one-component dental resin cement to at least one of a prosthetic and a tooth structure in dental treatment. For example, a surface of at least one of the tooth structure and the prosthetic is treated with the one-component dental pretreatment agent, and thereafter the prosthetic is bonded to the tooth with the one-component dental resin cement.

The A agent may be the one-component dental pretreatment agent. The one-component dental pretreatment agent contains an acidic substance. If necessary, the one-component dental pretreatment agent contains a non-acidic monomer, an oxidizing agent, a photopolymerization initiator, a filler, and another additive. The one-component dental pretreatment agent may not contain a non-acidic monomer and a filler.

When the A agent is the one-component dental pretreatment agent, the B agent is the one-component dental resin cement. The one-component dental resin cement contains a transition metal compound, a reducing agent, a non-acidic monomer, and a filler. If necessary, the one-component dental resin cement contains a photopolymerization initiator and another additive.

Alternatively, the A agent may be the one-component dental resin cement. The one-component dental resin cement contains an acidic substance, a non-acidic monomer, and a filler. If necessary, the one-component dental resin cement contains a photopolymerization initiator and another additive.

When the A agent is the one-component dental resin cement, the B agent is the one-component dental pretreatment agent. The one-component dental pretreatment agent contains a transition metal compound and a reducing agent. If necessary, the one-component dental pretreatment agent contains a non-acidic monomer, a photopolymerization initiator, a filler, and another additive. The one-component dental pretreatment agent may not contain a non-acidic monomer and a filler.

(2) The dental polymerization kit may contain a one-component dental pretreatment agent and a two-component dental resin cement. The one-component dental pretreatment agent is used, for example, to improve the adhesiveness of the two-component dental resin cement to at least one of a prosthetic and a tooth structure in dental treatment. For example, a surface of at least one of the tooth structure and the prosthetic is treated with the one-component dental pretreatment agent, and thereafter the prosthetics is bonded to the tooth with the two-component dental resin cement. The two-component dental resin cement includes a first agent and a second agent. The two-component dental resin cement may be used, for example, by mixing the first agent and the second agent together immediately before the use and applying the obtained mixture. Alternatively, the two-component dental resin cement may be used, for example, by applying the first agent and thereafter applying the second agent.

The A agent may be the one-component dental pretreatment agent. In this case, the one-component dental pretreatment agent contains an acidic substance. If necessary, the one-component dental pretreatment agent contains a non-acidic monomer, an oxidizing agent, a photopolymerization initiator, a filler, and another additive. The one-component dental pretreatment agent may not contain a non-acidic monomer and a filler.

When the A agent is the one-component dental pretreatment agent, the B agent may be the first agent of the two-component dental resin cement. In this case, the first agent of the two-component dental resin cement contains a transition metal compound and a reducing agent. If necessary, the first agent of the two-component dental resin cement contains a non-acidic monomer, a photopolymerization initiator, a filler, and another additive. The second agent of the two-component dental resin cement contains an arbitrary component. At least one of the first agent and second agent of the two-component dental resin cement contains a filler. At least one of the first agent and second agent of the two-component dental resin cement contains a non-acidic monomer.

Alternatively, the A agent may be the first agent of the two-component dental resin cement. In this case, the first agent of the two-component dental resin cement contains an acidic substance. If necessary, the first agent of the two-component dental resin cement contains a non-acidic monomer, a photopolymerization initiator, and another additive. One of the first agent and second agent of the two-component dental resin cement contains a non-acidic monomer. One of the first agent and second agent of the two-component dental resin cement contains a filler.

When the A agent is the first agent of the two-component dental resin cement, the B agent may be the one-component pretreatment agent. In this case, the one-component pretreatment agent contains a transition metal compound and a reducing agent. If necessary, the one-component pretreatment agent contains a non-acidic monomer, a photopolymerization initiator, a filler, and another additive. The one-component dental pretreatment agent may not contain a non-acidic monomer and a filler.

Alternatively, when the A agent is the first agent of the two-component dental resin cement, the B agent may be the second agent of the two-component dental resin cement. In this case, the second agent of the two-component dental resin cement contains a transition metal compound and a reducing agent. If necessary, the second agent of the two-component dental resin cement contains a non-acidic monomer, a photopolymerization initiator, a filler, and another additive. At least one of the first agent and second agent of the two-component dental resin cement contains a non-acidic monomer. At least one of the first agent and second agent of the two-component dental resin cement contains a filler. The one-component pretreatment agent contains an arbitrary component.

(3) The dental polymerization kit may contain a two-component dental pretreatment agent and a one-component dental resin cement. The two-component dental pretreatment agent is used, for example, to improve the adhesiveness of the dental resin cement to at least one of a prosthetic and a tooth structure in dental treatment. For example, the first agent and second agent of the two-component dental pretreatment agent are mixed together, and a surface of at least one of the tooth structure and the prosthetic is treated with the obtained mixture, and thereafter the prosthetic is bonded to the tooth with the one-component dental resin cement. Alternatively, for example, a surface of at least one of the tooth structure and the prosthetic is treated with the first agent of the two-component dental pretreatment agent, and thereafter the surface of at least one of the tooth structure and the prosthetic is treated with the second agent of the two-component dental pretreatment agent. Thereafter, for example, the prosthetic is bonded to the tooth with the one-component dental resin cement.

The A agent may be the first agent of the two-component dental pretreatment agent. In this case, the first agent of the two-component dental pretreatment agent contains an acidic substance. If necessary, the first agent of the two-component dental pretreatment agent contains a non-acidic monomer, an oxidizing agent, a photopolymerization initiator, a filler, and another additive.

When the A agent is the first agent of the two-component dental pretreatment agent, the B agent may be the second agent of the two-component dental pretreatment agent. In this case, the second agent of the two-component dental pretreatment agent contains a transition metal compound and a reducing agent. If necessary, the second agent of the two-component dental pretreatment agent contains a non-acidic monomer, a photopolymerization initiator, and another additive. The first agent and second agent of the two-component dental pretreatment agent may not contain a non-acidic monomer and a filler. In this case, the one-component dental resin cement contains a non-acidic monomer and a filler.

Alternatively, when the A agent is the first agent of the two-component dental pretreatment agent, the B agent may be the one-component dental resin cement. The one-component dental resin cement contains a transition metal compound, a reducing agent, a non-acidic monomer, and a filler. If necessary, the one-component dental resin cement contains a photopolymerization initiator and another additive. In this case, the second agent of the two-component dental pretreatment agent contains an arbitrary component.

Alternatively, the A agent may be the one-component dental resin cement. In this case, the one-component dental resin cement contains an acidic substance, a non-acidic monomer, and a filler. If necessary, the one-component dental resin cement contains a photopolymerization initiator and another additive.

When the A agent is the one-component dental resin cement, the B agent may be the first agent of the two-component dental pretreatment agent. In this case, the first agent of the two-component dental pretreatment agent contains a transition metal compound and a reducing agent. If necessary, the first agent of the two-component dental pretreatment agent contains a non-acidic monomer, an oxidizing agent, a photopolymerization initiator, a filler, and another additive. In this case, the second agent of the two-component dental pretreatment agent contains an arbitrary component. The first agent and second agent of the two-component dental pretreatment agent may not contain a non-acidic monomer.

(4) The dental polymerization kit may be a kit consisting of a two-component dental pretreatment agent and a two-component dental resin cement. The two-component dental pretreatment agent is used, for example, to improve the adhesiveness of the two-component dental resin cement to at least one of a prosthetic and a tooth structure in dental treatment. For example, the first agent and second agent of the two-component dental pretreatment agent are mixed together, and a surface of at least one of the tooth structure and the prosthetic is treated with the obtained mixture, and thereafter the prosthetic is bonded to the tooth with the two-component dental resin cement. Alternatively, for example, a surface of at least one of the tooth structure and the prosthetic is treated with the first agent of the two-component dental pretreatment agent, and thereafter the surface of at least one of the tooth structure and the prosthetic is treated with the second agent of the two-component dental pretreatment agent. Thereafter, for example, the prosthetics is bonded to the tooth with the two-component dental resin cement. The two-component dental resin cement may be used, for example, by mixing the first agent and the second agent together immediately before the use and applying the obtained mixture. Alternatively, the two-component dental resin cement may be used, for example, by applying the first agent and thereafter applying the second agent.

Alternatively, the A agent may be the first agent of the two-component dental pretreatment agent. The first agent of the two-component dental pretreatment agent contains an acidic substance. If necessary, the first agent of the two-component dental pretreatment agent contains a non-acidic monomer, an oxidizing agent, a photopolymerization initiator, a filler, and another additive.

When the A agent is the first agent of the two-component dental pretreatment agent, the B agent may be the second agent of the two-component dental pretreatment agent. The second agent of the two-component dental pretreatment agent contains a transition metal compound and a reducing agent. If necessary, the second agent of the two-component dental pretreatment agent contains a non-acidic monomer, a photopolymerization initiator, a filler, and another additive. The second agent of the two-component dental pretreatment agent may not contain a non-acidic monomer and a filler. In this case, at least one of the first agent and second agent of the two-component dental resin cement contains a non-acidic monomer. Further, at least one of the first agent and second agent of the two-component dental resin cement contains a filler.

Alternatively, when the A agent is the first agent of the two-component dental pretreatment agent, the B agent may be the first agent of the two-component dental resin cement. In this case, the first agent of the two-component dental resin cement contains a transition metal compound and a reducing agent. If necessary, the first agent of the two-component dental resin cement contains a non-acidic monomer, a photopolymerization initiator, a filler, and another additive. The second agent of the two-component dental pretreatment agent and the second agent of the two-component dental resin cement contain an arbitrary component. At least one of the first agent and second agent of the two-component dental resin cement contains a non-acidic monomer. At least one of the first agent and second agent of the two-component dental resin cement contains a filler.

Alternatively, the A agent may be the first agent of the two-component dental resin cement. In this case, the first agent of the two-component dental resin cement contains an acidic substance. If necessary, the first agent of the two-component dental resin cement contains a non-acidic monomer, a photopolymerization initiator, a filler, and another additive.

When the A agent is the first agent of the two-component dental resin cement, the B agent may be the second agent of the two-component dental resin cement. In this case, the second agent of the two-component dental resin cement contains a transition metal compound and a reducing agent. If necessary, the second agent of the two-component dental resin cement contains a non-acidic monomer, a photopolymerization initiator, a filler, and another additive. At least one of the first agent and second agent of the two-component dental resin cement contains a non-acidic monomer. Alternatively, at least one of the first agent and second agent of the two-component dental resin cement contains a filler. In this case, the first agent and second agent of the two-component dental pretreatment agent contain an arbitrary component.

Alternatively, when the A agent is the first agent of the two-component dental resin cement, the B agent may be the first agent of the two-component dental pretreatment agent. In this case, the first agent of the two-component dental pretreatment agent contains a transition metal compound and a reducing agent. If necessary, the first agent of the two-component dental pretreatment agent contains a non-acidic monomer, a photopolymerization initiator, a filler, and another additive. The first agent of the two-component dental pretreatment agent may not contain a non-acidic monomer and a filler. In this case, the second agent of the two-component dental resin cement and the second agent of the two-component dental pretreatment agent contain an arbitrary component. At least one of the first agent and second agent of the two-component dental resin cement contains a non-acidic monomer. At least one of the first agent and second agent of the two-component dental resin cement contains a filler.

(5) The dental polymerization kit may be a two-component dental self-adhesive composite resin. The two-component dental self-adhesive composite resin is used, for example, to fill the decayed part of a tooth in dental treatment. The definition of the self-adhesive is the same as that of the above-described self-adhesive of the two-component self-adhesive resin cement. The two-component dental self-adhesive composite resin contains an A agent and a B agent. The two-component dental self-adhesive composite resin may be used, for example, by mixing the A agent and the B agent together immediately before the use and applying the obtained mixture. Alternatively, the two-component dental self-adhesive composite resin may be used, for example, by applying the A agent and thereafter applying the B agent.

The A agent contains an acidic substance. If necessary, the A agent contains a non-acidic monomer, an oxidizing agent, a photopolymerization initiator, a filler, and another additive. The B agent contains a transition metal compound and a reducing agent. If necessary, the B agent contains a non-acidic monomer, a photopolymerization initiator, a filler, and another additive. At least one of the A agent and the B agent contains a non-acidic monomer. Further, at least one of the A agent and the B agent contains a filler.

(6) The dental polymerization kit may contain a one-component dental pretreatment agent and a one-component dental composite resin. The one-component dental pretreatment agent is used, for example, to improve the adhesiveness of the one-component dental composite resin to a tooth structure in dental treatment. For example, a surface of the tooth is treated with the one-component dental pretreatment agent, and thereafter the decayed part of the tooth is filled with the one-component dental composite resin.

The A agent may be the one-component dental pretreatment agent. In this case, the one-component dental pretreatment agent contains an acidic substance. If necessary, the one-component dental pretreatment agent contains a non-acidic monomer, an oxidizing agent, a photopolymerization initiator, a filler, and another additive. The one-component dental pretreatment agent may not contain a non-acidic monomer and a filler.

When the A agent is the one-component dental pretreatment agent, the B agent may be the one-component dental composite resin. In this case, the one-component dental composite resin contains a transition metal compound, a reducing agent, a non-acidic monomer, and a filler. If necessary, the one-component dental composite resin contains a photopolymerization initiator and another additive.

Alternatively, the A agent may be the one-component dental composite resin. The one-component dental composite resin contains an acidic substance, a non-acidic monomer, and a filler. If necessary, the one-component dental composite resin contains a photopolymerization initiator and another additive.

When the A agent is the one-component dental composite resin, the B agent may the one-component dental pretreatment agent. The one-component dental pretreatment agent contains a transition metal compound and a reducing agent. If necessary, the one-component pretreatment agent contains a non-acidic monomer, a photopolymerization initiator, a filler, and another additive. The one-component dental pretreatment agent may not contain a non-acidic monomer and a filler.

(7) The dental polymerization kit may be a kit consisting of a one-component dental pretreatment agent and a two-component dental composite resin. The one-component dental pretreatment agent is used, for example, to improve the adhesiveness of the two-component dental composite resin to a tooth structure in dental treatment. For example, a surface of the tooth is treated with the one-component dental pretreatment agent, and thereafter the decayed part of the tooth is filled with the two-component dental composite resin. The two-component dental composite resin may be used, for example, by mixing the first agent and the second agent together immediately before the use and applying the obtained mixture. Alternatively, the two-component dental composite resin may be used, for example, by applying the first agent and thereafter applying the second agent.

The A agent may be a one-component dental pretreatment agent. The one-component dental pretreatment agent contains an acidic substance. If necessary, the one-component dental pretreatment agent contains a non-acidic monomer, an oxidizing agent, a photopolymerization initiator, a filler, and another additive.

When the A agent is the one-component dental pretreatment agent, the B agent may be the first agent of the two-component dental resin cement. The first agent of the two-component dental resin cement contains a transition metal compound and a reducing agent. If necessary, the first agent of the two-component dental composite resin contains a non-acidic monomer, a photopolymerization initiator, a filler, and another additive. In this case, the second agent of the two-component dental composite resin contains an arbitrary component. At least one of the first agent and second agent of the two-component dental composite resin contains a non-acidic monomer. At least one of the first agent and second agent of the two-component dental composite resin contains a filler.

Alternatively, the A agent may be the first agent of the two-component dental composite resin. In this case, the first agent of the two-component dental composite resin contains an acidic substance. If necessary, the first agent of the two-component dental resin cement contains a non-acidic monomer, a photopolymerization initiator, a filler, and another additive.

When the A agent is the first agent of the two-component dental composite resin, the B agent may be the one-component dental pretreatment agent. The one-component dental pretreatment agent contains a transition metal compound and a reducing agent. If necessary, the one-component dental pretreatment agent contains a non-acidic monomer, a photopolymerization initiator, a filler, and another additive. The one-component dental pretreatment agent may not contanin a non-acidic monomer and a filler. The second agent of the two-component dental composite resin contains an arbitrary component. At least one of the first agent and second agent of the two-component dental composite resin contains a non-acidic monomer. Further, at least one of the first agent and second agent of the two-component dental composite resin contains a filler.

Alternatively, when the A agent is the first agent of the two-component dental composite resin, the B agent may be the second agent of the two-component dental composite resin. The second agent of the two-component dental composite resin contains a transition metal compound and a reducing agent. If necessary, the second agent of the two-component dental composite resin contains a non-acidic monomer, a photopolymerization initiator, and another additive. At least one of the first agent and second agent of the two-component dental composite resin contains a non-acidic monomer. At least one of the first agent and second agent of the two-component dental composite resin contains a filler. In this case, the one-component pretreatment agent contains an arbitrary component.

(8) The dental polymerization kit may include a two-component dental pretreatment agent and a one-component dental composite resin. The two-component dental pretreatment agent is used, for example, to improve the adhesiveness of the one-component dental composite resin to a tooth structure in dental treatment. For example, a surface of the tooth is treated with the two-component dental pretreatment agent, and thereafter the decayed part of the tooth is filled with the one-component dental composite resin. The two-component dental pretreatment agent may be used, for example, by mixing the first agent and the second agent together immediately before the use and applying the obtained mixture. Alternatively, the two-component dental pretreatment agent may be used, for example, by applying the first agent and thereafter applying the second agent.

The A agent may be the first agent of the two-component dental pretreatment agent. In this case, the first agent of the two-component dental pretreatment agent contains an acidic substance. If necessary, the first agent of the two-component dental pretreatment agent contains a non-acidic monomer, an oxidizing agent, a photopolymerization initiator, a filler, and another additive.

When the A agent is the first agent of the two-component dental pretreatment agent, the B agent may be the second agent of the two-component dental pretreatment agent. The second agent of the two-component dental pretreatment agent contains a transition metal compound and a reducing agent. If necessary, the second agent of the two-component dental pretreatment agent contains a non-acidic monomer, a photopolymerization initiator, a filler, and another additive. The first agent and second agent of the two-component dental pretreatment agent may not contain a non-acidic monomer and a filler. The one-component dental composite resin contains a non-acidic monomer.

Alternatively, when the A agent is the first agent of the two-component dental pretreatment agent, the B agent may be the one-component dental composite resin. The one-component dental composite resin contains a transition metal compound, a reducing agent, a non-acidic monomer, and a filler. If necessary, the one-component dental composite resin contains a photopolymerization initiator and another additive. In this case, the second agent of the two-component dental pretreatment agent contains an arbitrary component.

Alternatively, the A agent may be the one-component dental composite resin. In this case, the one-component dental composite resin contains an acidic substance, a non-acidic monomer, and a filler. If necessary, the one-component dental composite resin contains a photopolymerization initiator and another additive.

When the A agent is the one-component dental composite resin, the B agent may be the first agent of the two-component dental pretreatment agent. The first agent of the two-component dental pretreatment agent contains a transition metal compound and a reducing agent. If necessary, the first agent of the two-component dental pretreatment agent contains a non-acidic monomer, an oxidizing agent, a photopolymerization initiator, and another additive. In this case, the second agent of the two-component dental pretreatment agent contains an arbitrary component. The first agent and second agent of the two-component dental pretreatment agent may not contain a non-acidic monomer and a filler.

(9) The dental polymerization kit may include a two-component dental pretreatment agent and a two-component dental composite resin. The two-component dental pretreatment agent is used, for example, to improve the adhesiveness of the two-component dental composite resin to a tooth structure in dental treatment. For example, a surface of the tooth is treated with the two-component dental pretreatment agent, and thereafter the decayed part of the tooth is filled with the two-component dental composite resin. The two-component dental pretreatment agent may be used, for example, by mixing the first agent and the second agent together immediately before the use and applying the obtained mixture. Alternatively, the two-component dental pretreatment agent may be used, for example, by applying the first agent and thereafter applying the second agent. Further, the two-component dental composite resin may be used, for example, by mixing the first agent and the second agent together immediately before the use and applying the obtained mixture. Alternatively, the the two-component dental composite resin may be used, for example, by applying the first agent and thereafter applying the second agent.

When the A agent may be the first agent of the two-component dental pretreatment agent. The first agent of the two-component dental pretreatment agent contains an acidic substance. If necessary, the first agent of the two-component dental pretreatment agent contains a non-acidic monomer, an oxidizing agent, a photopolymerization initiator, and another additive.

When the A agent is the first agent of the two-component dental pretreatment agent, the B agent may be the second agent of the two-component dental pretreatment agent. The second agent of the two-component dental pretreatment agent contains a transition metal compound and a reducing agent. If necessary, the second agent of the two-component dental pretreatment agent contains a non-acidic monomer, a photopolymerization initiator, and another additive. The first agent and second agent of the two-component dental pretreatment agent may not contain a non-acidic monomer and a filler. In this case, the first agent and second agent of the two-component dental composite resin contain an arbitrary component. At least one of the first agent and second agent of the two-component dental composite resin contains a non-acidic monomer. At least one of the first agent and second agent of the two-component dental composite resin contains a filler.

Alternatively, when the A agent is the first agent of the two-component dental pretreatment agent, the B agent may be the first agent of the two-component dental composite resin. The first agent of the two-component dental composite resin contains a transition metal compound and a reducing agent. If necessary, the first agent of the two-component dental composite resin contains a non-acidic monomer, a photopolymerization initiator, a filler, and another additive. In this case, the second agent of the two-component dental pretreatment agent and the second agent of the two-component dental composite resin contain an arbitrary component. At least one of the first agent and second agent of the two-component dental composite resin contains a non-acidic monomer. At least one of the first agent and second agent of the two-component dental composite resin contains a filler.

Alternatively, the A agent may be the first agent of the two-component dental composite resin. In this case, the first agent of the two-component dental composite resin contains an acidic substance. If necessary, the first agent of the two-component dental composite resin contains a non-acidic monomer, a photopolymerization initiator, and another additive.

When the A agent is the first agent of the two-component dental composite resin, the B agent is the first agent of the two-component dental pretreatment agent. The first agent of the two-component dental pretreatment agent contains a transition metal compound and a reducing agent. If necessary, the first agent of the two-component dental pretreatment agent contains a non-acidic monomer, a photopolymerization initiator, a filler, and another additive. The second agent of the two-component dental pretreatment agent and the second agent of the two-component dental composite resin contain an arbitrary component. At least one of the first agent and second agent of the two-component dental composite resin contains a non-acidic monomer. At least one of the first agent and second agent of the two-component dental composite resin contains a filler.

Alternatively, when the A agent is the first agent of the two-component dental composite resin, the B agent may be the second agent of the two-component dental composite resin. The second agent of the two-component dental composite resin contains a transition metal compound and a reducing agent. If necessary, the second agent of the two-component dental composite resin contains a non-acidic monomer, a photopolymerization initiator, and another additive. The first agent and second agent of the two-component dental pretreatment agent contain an arbitrary component. At least one of the first agent and second agent of the two-component dental composite resin contains a non-acidic monomer. At least one of the first agent and second agent of the two-component dental composite resin contains a filler.

(10) The dental polymerization kit may be a two-component dental coating agent. The two-component dental coating agent is used, for example, to coat a surface of a tooth structure in dental treatment. The two-component dental coating agent contains an A agent and a B agent. The two-component dental coating agent may be used, for example, by mixing the A agent and the B agent together immediately before the use and applying the obtained mixture. Alternatively, the two-component dental coating agent may be used, for example, by applying the A agent and thereafter applying the B agent.

The A agent contains an acidic substance. If necessary, the A agent contains a non-acidic monomer, an oxidizing agent, a photopolymerization initiator, a filler, and another additive. The B agent contains a transition metal compound and a reducing agent. If necessary, the B agent contains a non-acidic monomer, a photopolymerization initiator, a filler, and another additive. At least one of the A agent and the B agent contains a non-acidic monomer. The A agent and B agent may not contain a filler.

### Example

The present invention is more specifically described below with reference to Examples and Comparative Examples. The present invention is not limited to Examples in any way. The specific numeral values used in the description below, such as mixing ratios (content ratios), physical property values, and parameters, can be replaced with the corresponding mixing ratios (content ratios), physical property values, and parameters in the above-described "DESCRIPTION OF THE EMBODIMENTS", including the upper limit values (numeral values defined with "or less", and "less than") or the lower limit values (numeral values defined with "or more", and "more than").

### 1. Preparation of A agent and B agent

According to Tables 1 to 8, the components was mixed together, thereby preparing the A agent and B agent of each dental polymerization kit.

In each Embodiment, the transition metal compound was not dissolved in the B agent and solid in the B agent.

On the other hand, in each of Comparative Examples, the transition metal compound other than copper(II) acetate monohydrate and copper(II) chloride dihydrate were dissolved in the A agent or the B agent and cured the A agent or the B agent within 24 hours. Further, the copper(II) acetate·monohydrate and copper(II) chloride·dihydrate were gradually dissolved in the A agent or the B agent due to the moisture absorption from wet air and cured the A agent or the B agent within 24 hours.

The meanings of the abbreviations in Table 1 to 8 were listed below.

Bis-GMA: 2,2-bis[4-(3-(meth)acryloyl oxy-2-hydroxypropoxy)phenyl] propane
HEMA: 2-hydroxyethylmethacrylate
UDMA: [2,2,4-trimethylhexamethylenebis(2-carbamoyl oxyethyl)]dimethacrylate
D-2.6E: ethylene oxide-modified bisphenol A di(meth)acrylate (the addition mole number of ethylene oxide: 2.6)
NPGDMA: neopentyl glycol dimethacrylate
MDP: 10-methacryloyl oxy decyl acid phosphate
4-MET: 4-methacryloyl oxyethyl trimellitic acid
PA: 85% phosphoric acid
HCI: 35 to 37% muriatic acid
CQ: camphorquinone
EDAB: ethyl 4-(dimethylamino)benzoate
DMPT: N,N-dimethyl-p-toluidine
SPG: sodium N-phenylglycine (particulates, a particle size of 53µm or less)
STS: sodium p-toluenesulfinate (particulates, a particle size of 63µm or less)
TMTU: trimethylthiourea
PTU: (2-pyridyl)thiourea
BPB: t-butylperoxybenzoate
CHP: cumene hydroperoxide
BI-Cu: benzimidazole copper (particulates, a particle size of 20µm or less)
PA-Cu: 1-3-propanediamine copper (particulates, a particle size of 20µm or less)
BS-Cu: benzothiazole copper (particulates, a particle size of 20µm or less)
TU-Cu: thiourea copper (particulates, a particle size of 20µm or less)
Cu(OH)₂: copper(II) hydroxide (particulates, a particle size of 20µm or less)
BCuC: basic copper(II) carbonate (particulates, a particle size of 20µm or less)
VO: vanadium(V) oxide (particulates, a particle size of 20µm or less)
CuAc·H₂O: copper(II) acetate monohydrate (particulates, a particle size of 20µm or less)
CuCl₂·2H₂O: copper(II) chloride ·dihydrate (particulates, a particle size of 20µm or less)
CuAA: copper(II) acetylacetonate (particulates, a particle size of 20µm or less)
VOAA: vanadium(IV) oxyacetylacetonate (particulates, a particle size of 20µm or less)
BHT: dibutylhydroxytoluene
R812: fumed silica manufactured by AEROSIL
GM8235: (silanized) barium glass filler manufactured by SCHOTT

### 2. Evaluations

### (1) Preservation Stability

The A agent and B agent of the dental polymerization kit of each of Examples and Comparative Examples were filled in a light-blocking double-barrel syringe. The time 24 hours after the syringe had been left at 23°C and a pressure of one atmosphere under wet air was referred to as "controlled", and the time 15 days after was referred to as "after storage". The preservation stability of the dental polymerization kit was evaluated using the following criteria. The results are shown in Tables 1 to 8.
Good: The increase in viscosity and the curing were not confirmed.
Bad: The agents were cured.

### (2) Initial curing time

Using the dental polymerization kit of each of Examples and Comparative Examples, differential scanning calorimetry was carried out to measure the length of time [min: sec] until the polymerization heat reached its peak, thereby measuring the adhesiveness at the time (controlled) 24 hours after the syringe had been left at 23°C and a pressure of one atmosphere under wet air and the time (after storage) 15 days after the syringe had been left at 23°C and a pressure of one atmosphere under wet air. The results are shown as initial curing times in Tables 1 to 8.

### (3) Tensile Strength

Using the dental polymerization kit of each of Examples and Comparative Examples, the adhesiveness at the time (controlled) 24 hours after the syringe had been left at 23°C and a pressure of one atmosphere under wet air and the time (after storage) 15 days after the syringe had been left at 23°C and a pressure of one atmosphere under wet air were evaluated with the following method. The results are shown in Tables 1 to 8.

### (3-1) Adhesiveness with Pretreatment (with Pretreatment Agent)

A bovine tooth was polished with emery paper (P180) while water was being injected, thereby forming a flat dentin surface. A tape having a 4.8 mm-diameter hole was bonded to the dentin surface, thereby defining an adhesive surface. An appropriate pretreatment agent was applied to the adhesive surface exposed at the hole of the tape and air blown until the coating film did not move. Immediately after that, a stainless-steel rod was bonded to the coated adhesive surface with the dental polymerization kit of each of Examples 1 to 14 and Comparative Examples 1 to 20, and left to stand at 25°C and at a pressure of one atmosphere under wet air for 30 minutes, and immersed in 37°C water. 24 hours later, the bovine tooth was taken out of the water, and the tensile strength of the dental polymerization kit was measured at a test rate of 2 mm/min.

### (3-2) Adhesiveness without Pretreatment (without Pretreatment agent)

A bovine tooth was polished with emery paper (P180) while water was being injected, thereby forming a flat dentin surface. A tape having a 4.8 mm-diameter hole was bonded to the dentin surface, thereby defining an adhesive surface. A stainless-steel rod was bonded to the adhesive surface exposed at the hole of the tape with the dental polymerization kit of each of Examples 15 to 17 and Comparative Examples 21 to 23, and left to stand at 25°C and at a pressure of one atmosphere under wet air for 30 minutes, and immersed in 37°C water. 24 hours later, the bovine tooth was taken out of the water, and the tensile strength of the dental polymerization kit was measured at a test rate of 2 mm/min.

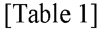

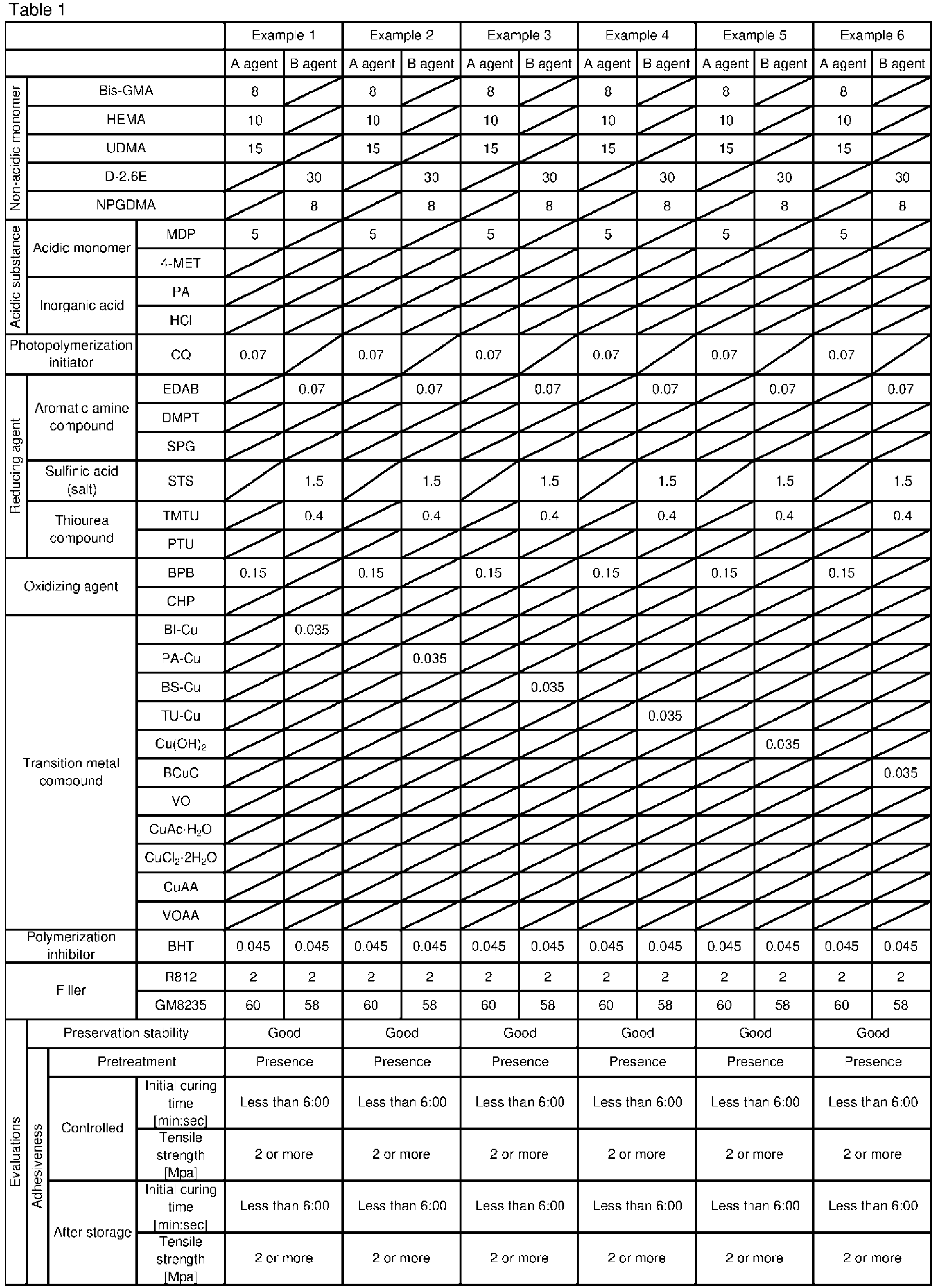

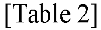

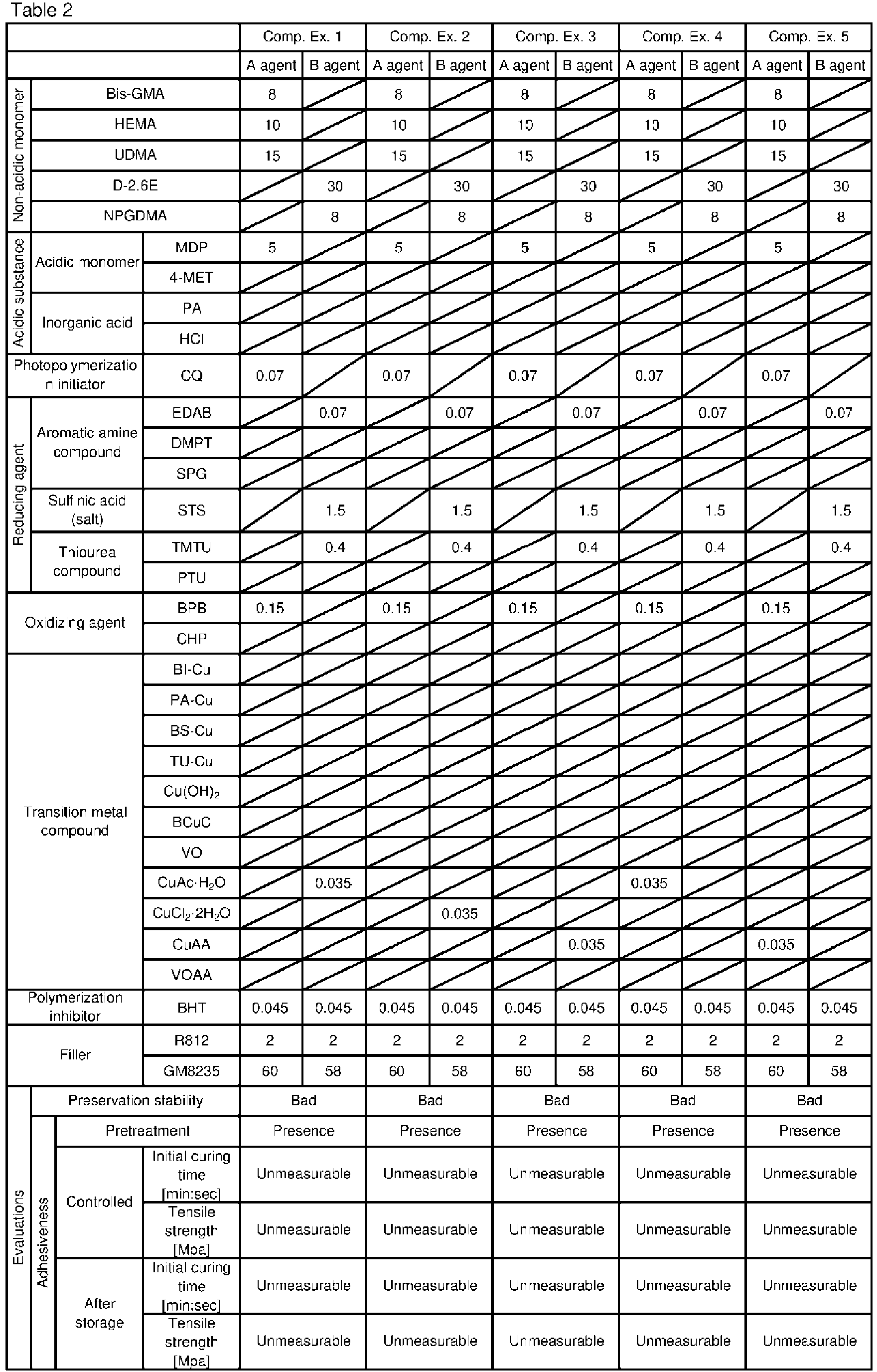

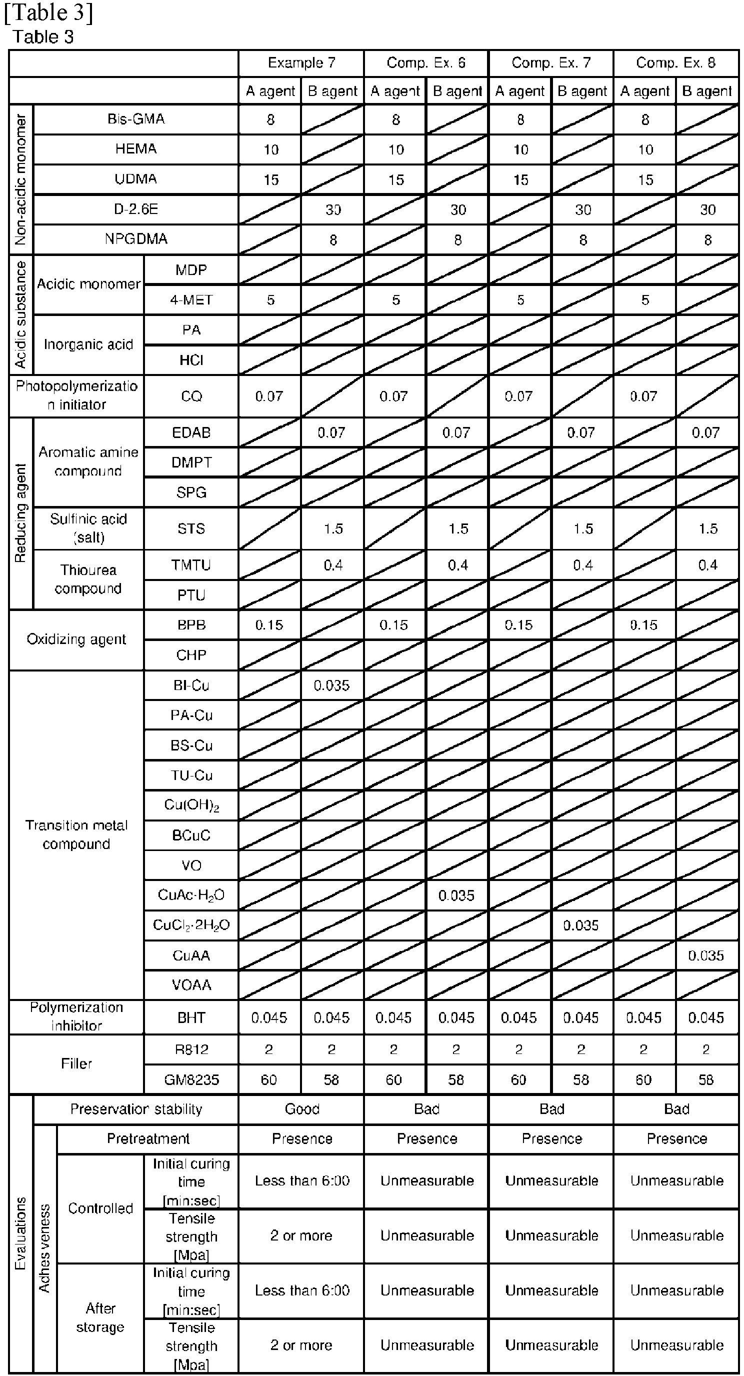

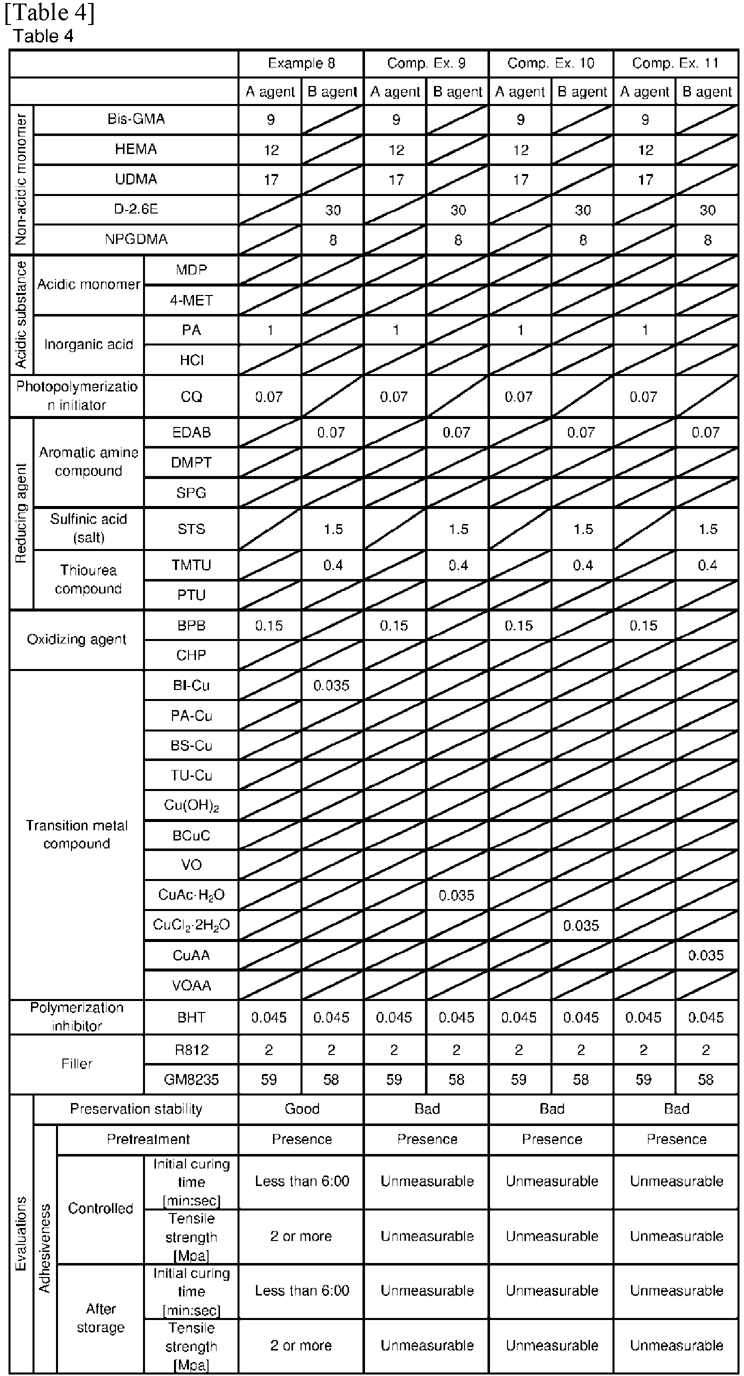

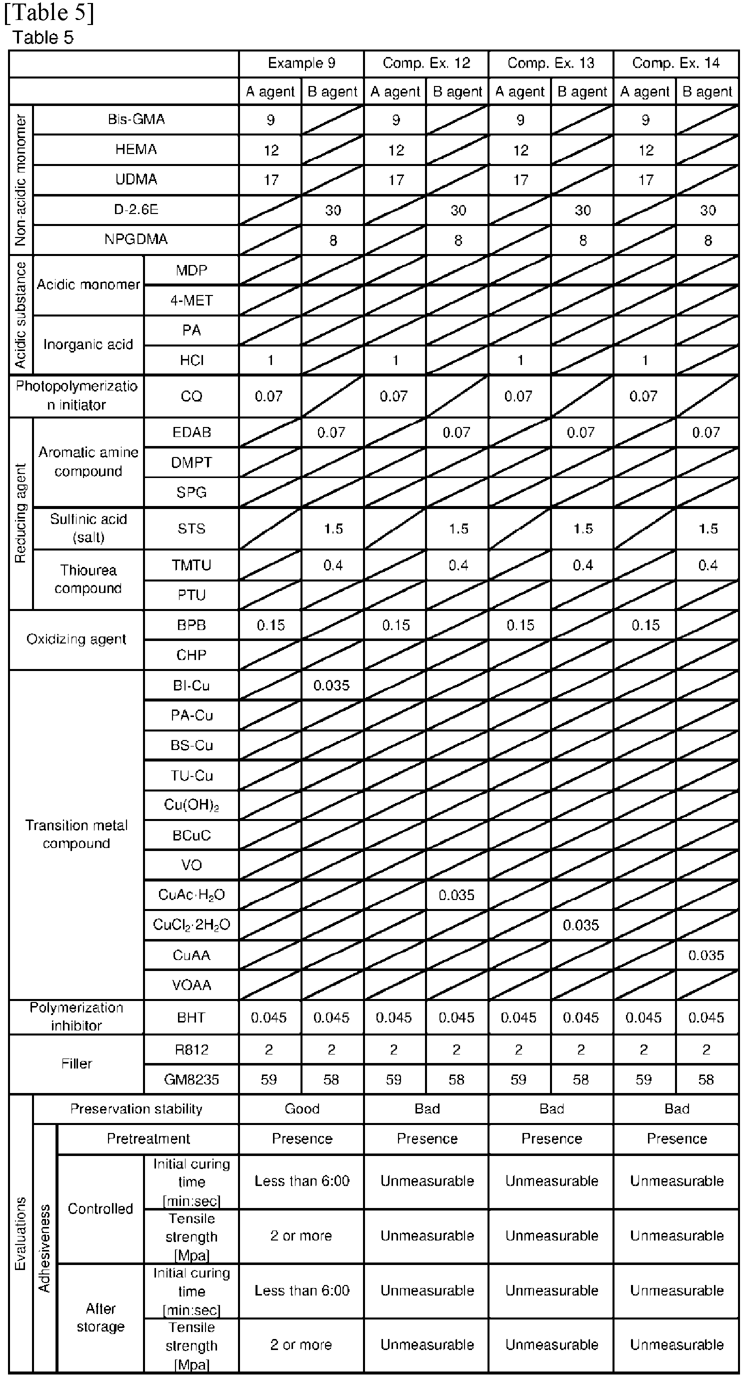

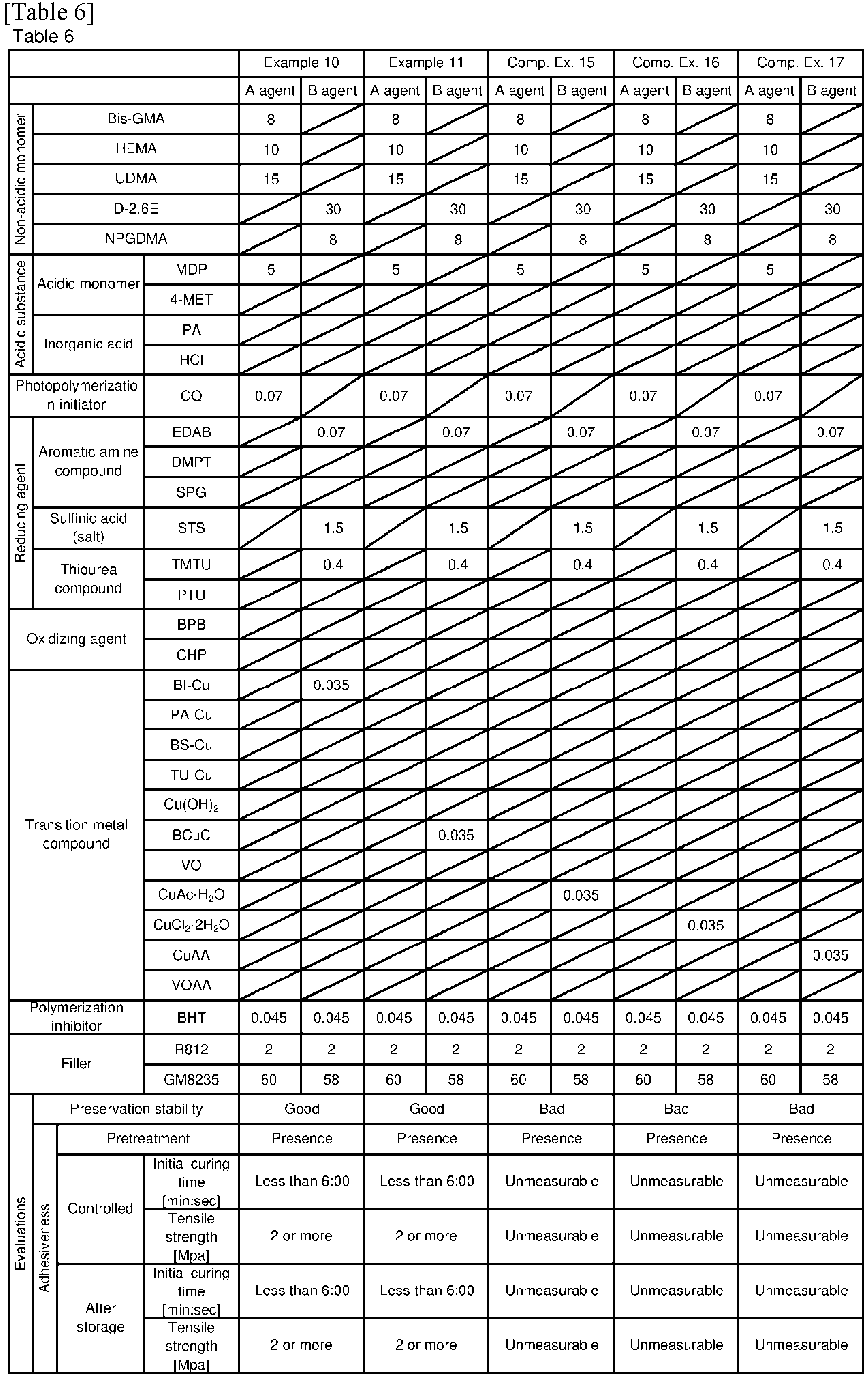

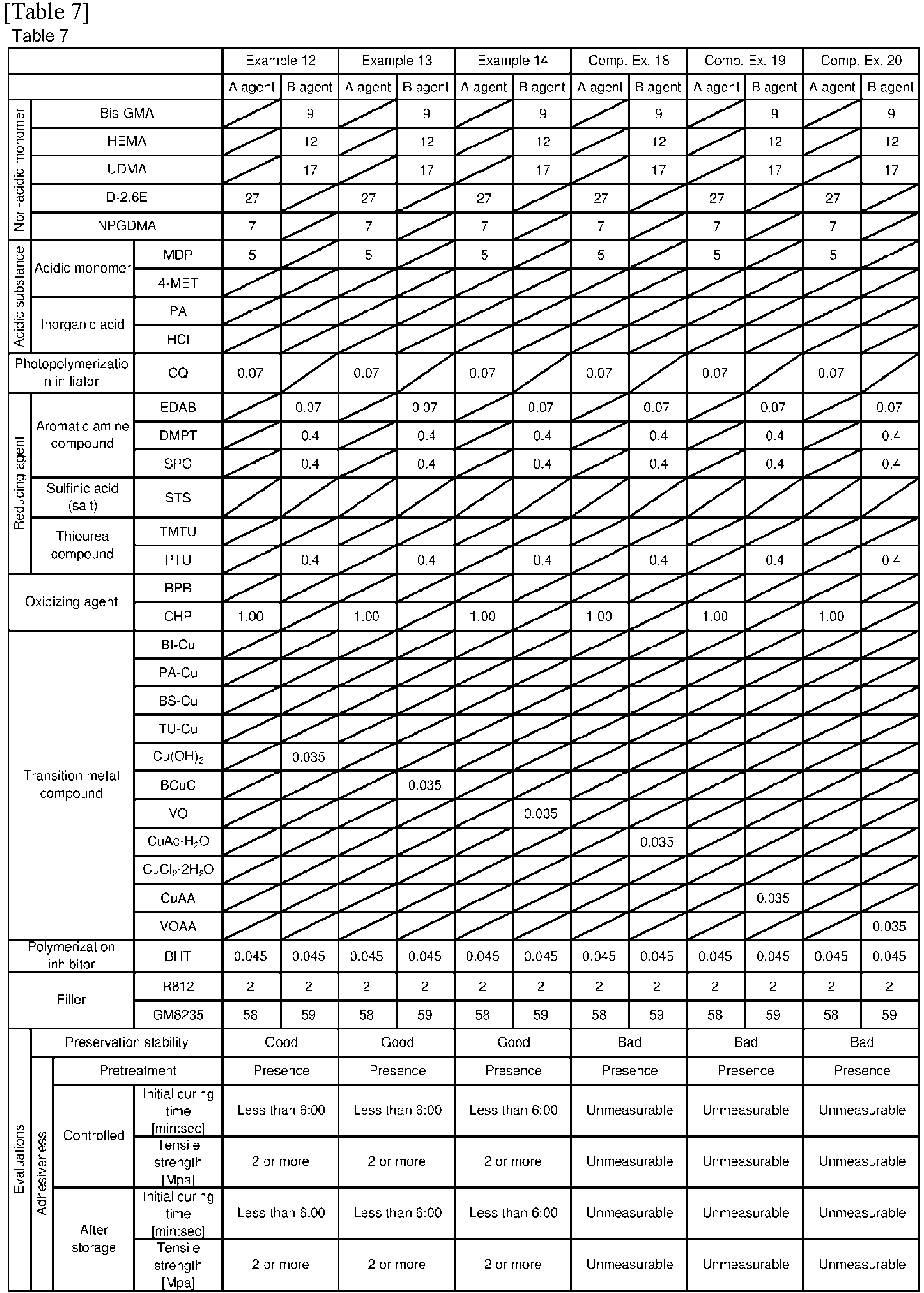

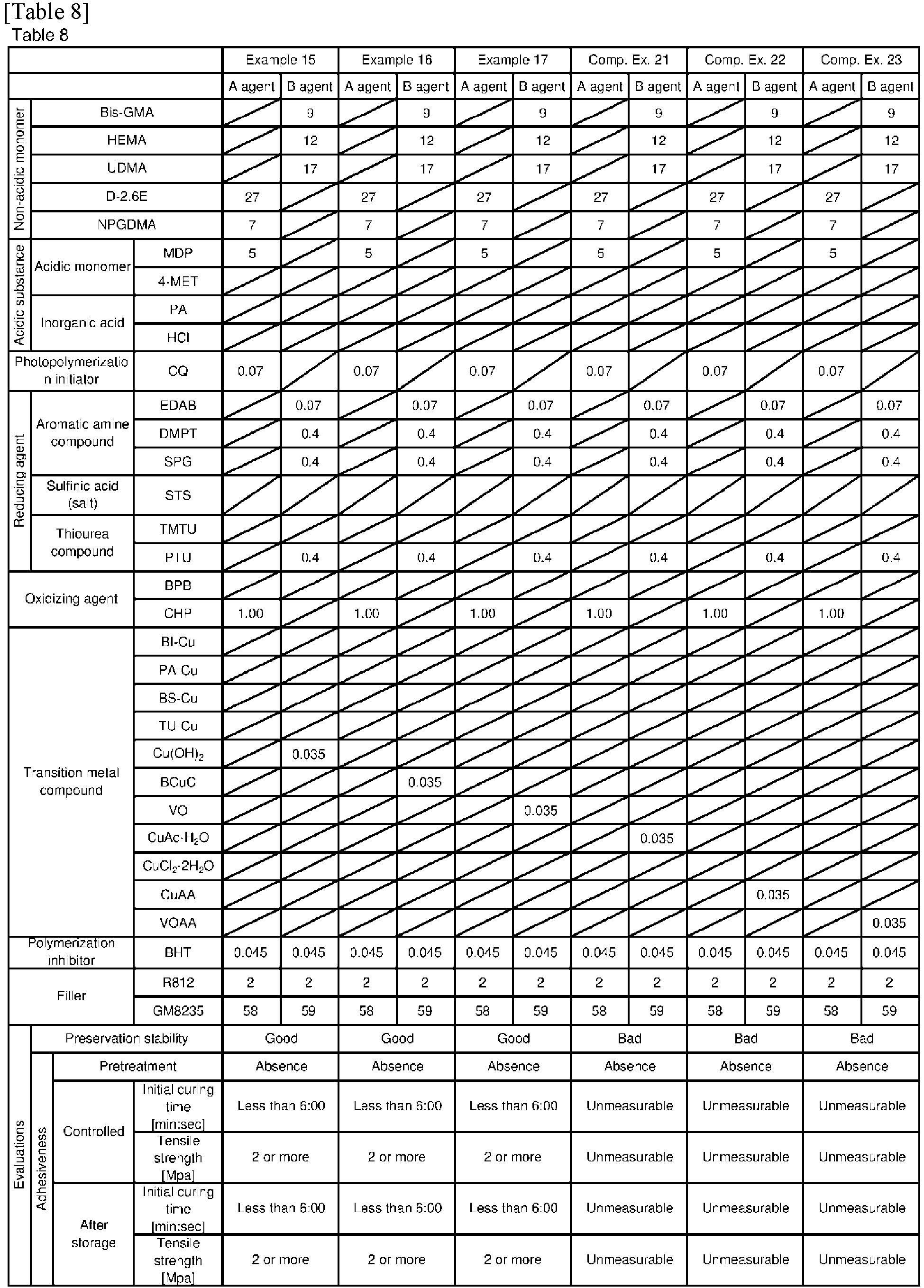

The results in Table 1 to 8 show that the dental polymerization kit of the present invention has excellent preservation stability, and the initial curing time thereof is short (within 6 minutes). Further, they show that the dental polymerization kit of the present invention has excellent adhesiveness to a tooth structure (a tensile strength of 2 MPa or more). Therefore, the dental polymerization kit of the present invention is useful as a dental material.

While the illustrative embodiments of the present invention are provided in the above description, such is for illustrative purpose only and it is not to be construed as limiting in any manner. Modification and variation of the present invention that will be obvious to those skilled in the art is to be covered by the following claims.

### Industrial Applicability

The dental polymerization kit of the present invention is used for dental treatments.

## Claims

1. A dental polymerization kit used to polymerize a non-acidic monomer having no acidic group and having an ethylenic unsaturated group in dental treatment, the dental polymerization kit comprising:
an A agent containing an acidic substance; and
a B agent containing a transition metal compound and a reducing agent and being in the form of a liquid or a paste,
wherein the transition metal compound is solid in the B agent and soluble in the acidic substance.

2. The dental polymerization kit according to claim 1, wherein the A agent further contains an oxidizing agent.

3. The dental polymerization kit according to claim 1, wherein the acidic substance is an acidic monomer having an acidic group and an ethylenic unsaturated group.

4. The dental polymerization kit according to claim 1, wherein at least one of the A agent and the B agent contains the non-acidic monomer.

5. The dental polymerization kit according to claim 1, wherein a ratio of the transition metal compound in the B agent is 0.0001% by mass or more.
